# FASCICULE DE BREVET EUROPEEN

(11) **EP 1 343 486 B1**
(45) Date de publication et mention de la délivrance du brevet: **13.09.2006**
(21) Numéro de dépôt: 01996374.3
(22) Date de dépôt: 14.11.2001
(51) Int. Cl.: A61K 31/045, A61K 31/56, A61P 17/06, A61P 37/08

(54) **COMPOSITION PHARMACEUTIQUE OU COSMETIQUE ET UTILISATION D'UN INHIBITEUR DE PKC AVEC UN INHIBITEUR DE MMP POUR INHIBER LA MIGRATION DES CELLULES DE LANGERHANS**
PHARMAZEUTISCHE ODER KOSMETISCHE ZUSAMMENSETZUNG UND VERWENDUNG EINES PKC-INHIBITORS MIT EINEM MMP-INHIBITOR ZUR HEMMUNG DER MIGRATION VON LANGERHANS-ZELLEN
PHARMACEUTICAL OR COSMETIC COMPOSITION AND USE OF A PKC INHIBITOR WITH AN MMP INHIBITOR FOR INHIBITING LANGERHANS' CELL MIGRATION

(30) Priorité: 14.11.2000 FR 0014607
(43) Date de publication de la demande: 17.09.2003
(73) Titulaire: Laboratoires Expanscience, 92400 Courbevoie (FR)
(72) Inventeur: MSIKA, Philippe, F-75018 Paris (FR); PICCARD, Nathalie, 38120 Saint-Egreve (FR); PICCIRILLI, Antoine, F-28230 Epernon (FR)
(74) Mandataire: Warcoin, Jacques
(86) Numéro de dépôt international: PCT/FR2001/003568
(87) Numéro de publication internationale: WO 2002/040004

(56) Documents cités:
- WO-A-00/03734
- WO-A-00/62789
- WO-A-93/23075
- WO-A-98/43959
- US-A- 5 891 906
- US-A- 5 981 491
- US-A- 6 040 152
- HALLIDAY G M ET AL: "Protein kinase C transduces the signal for Langerhans' cell migration from the epidermis." IMMUNOLOGY, vol. 79, no. 4, 1993, pages 621-626, XP001011467 ISSN: 0019-2805
- BHEEKHA-ESCURA R ET AL: "PHARMACOLOGIC REGULATION OF HISTAMINE RELEASE BY THE HUMAN RECOMBINANT HISTAMINE-RELEASING FACTOR" JOURNAL OF ALLERGY AND CLINICAL IMMUNOLOGY,MOSBY - YEARLY BOOK, INC,US, vol. 103, no. 5, mai 1999 (1999-05), pages 937-943, XP000991205 ISSN: 0091-6749
- GISHER G J ET AL: "DIFFERENTIAL EXPRESSION OF CONVENTIONAL AND NONCONVENTIONAL PROTEIN KINASE C ISOZYMES IN NORMAL AND PSORIATIC SKIN" JOURNAL OF INVESTIGATIVE DERMATOLOGY, vol. 98, no. 4, 1992, page 635 XP001011469 ISSN: 0022-202X
- BURNHAM K ET AL: "Requirements for Langerhans' cell depletion following in vitro exposure of murine skin to ultraviolet-B." IMMUNOLOGY, vol. 79, no. 4, 1993, pages 627-632, XP001011468 ISSN: 0019-2805
- LIESVELD JANE L ET AL: "Studies of progenitor cell transendothelial migration: Effects of cytokine mediators, signal transduction inhibitors, and gelatinases." BLOOD, vol. 96, no. 11 Part 2, 16 novembre 2000 (2000-11-16), page 151b XP008000675 42nd Annual Meeting of the American Society of Hematology;San Francisco, California, USA; December 01-05, 2000 ISSN: 0006-4971
- CHANG Y C ET AL: "Regulation of matrix metalloproteinase-2 production by cytokines and pharmacological agents in human pulp cell cultures." JOURNAL OF ENDODONTICS, (2001 NOV) 27 (11) 679-82. , XP008000633

## Description

La présente invention concerne une composition pharmaceutique ou cosmétique ainsi que l'utilisation d'au moins un composé actif pour inhiber la migration des cellules impliquées dans la réponse immunitaire et/ou inflammatoire et/ou irritative telles que les dendrocytes dermiques, les monocytes, les lymphocytes, et plus particulièrement les cellules de Langerhans.

Une des principales fonction de la peau est la protection de l'organisme contre des agressions du milieu extérieur. Cette protection est assurée en grande partie grâce à la coopération de cellules présentes dans la peau, cellules qui sont capables en présence d'un agent nocif de générer une réponse inflammatoire et/ou immunitaire dirigée contre l'agent nocif. Ce sont les cellules dendritiques, les cellules de Langerhans (CL) de J'épiderme, et dendrocytes dermiques, les monocytes, les lymphocytes, les kératinocytes, les mastocytes, et les cellules endothéliales vasculaires.

Les CL sont des cellules dendritiques issues de la moelle épinière et qui résident dans les tissus non lymphoïdes tels que la peau et les muqueuses (bouche, poumon, vessie, rectum, vagin). Dans la peau, les CL s'intercalent entre les kératinocytes épidermiques en position suprabasale. Sur le plan ultrastructural, elles sont caractérisées par la présence d'un organite spécifique d'origine membranaire, le granule de Birbeck. Sur le plan immunohistochimique, les CL expriment notamment la molécule CD1a et les molécules du Complexe Majeur d'Histocompatibilité de classe II.

Les CL jouent un rôle déterminant dans l'immunité, en tant que cellules présentatrices de l'antigène. En effet, des expériences menées chez la souris démontrent que les CL capturent les antigènes présents au niveau de l'épiderme et migrent vers les tissus lymphoïdes drainants la peau, où elles présentent l'antigène aux cellules T. L'initiation de la réponse immune cutanée dépend de la capacité des CL à quitter l'épiderme pour migrer jusqu'aux ganglions proximaux. Différents facteurs peuvent influencer cette migration : l'expression de molécules d'adhérence, les protéines de la matrice extracellulaire, des haptènes, des cytokines, etc. Néanmoins, les mécanismes impliqués dans la migration des CL ne sont pas totalement encore élucidés. En particulier, avant d'atteindre les ganglions lymphatiques, les CL doivent non seulement traverser la jonction dermo-épidermique (JDE) mais également se frayer un chemin au travers de la matrice extracellulaire (MEC) dermique. La JDE est principalement composés de laminine 5, de collagène de type IV et VII, de nidogène et de perlecan. La MEC qui entoure les fibroblastes du derme contient essentiellement des collagènes de type 1 et III.

Des pathologies de type dermatologique peuvent également être observées comme résultant de la migration des CL suite à la capture d'un antigène de surface.

Dans l'eczéma atopique, les CL sont capables de fixer des IgE en surface et d'induire une réponse immunitaire pathologique.

Dans l'eczéma de contact, les CL jouent un rôle central puisqu'elles captent et traitent l'antigène avant de le présenter aux lymphocytes T. Celui-ci va le garder en mémoire et la réaction immunitaire sera déclenchée au deuxième contact.

Compte tenu de ce qui précède, il était donc hautement souhaitable de pouvoir modifier la capacité migratoire des CL, pour tenter de modifier l'induction de la réaction immune et /ou inflammatoire.

On a maintenant trouvé de manière tout à fait surprenante et inattendue que certains composés permettent d'inhiber de manière spectaculaire la migration des cellule de Langerhans induite par la présence d'un agent allergène.

La présente invention concerne ainsi une composition pharmaceutique ou cosmétique, caractérisée en ce qu'elle comprend une association d'un composé actif pour inhiber la migration des cellules de Langerhans et inhibiteur des protéines kinases C (PKC) qui est la sphingosine ou la phytosphingosino, et un composé actif pour inhiber la migration des cellules de Langerhans et inhibiteur des métalloprotéases matricielles (MMPs) qui est un extrait peptidique de lupin et au moins un excipient pharmaceutiquement ou cosmétiquement acceptable.

Par « protéines kinases C » ou « PKC », on entend selon l'invention les enzymes qui catalysent une réaction de phosphorylation sur un substrat cellulaire.

Lorsqu'elles sont activées, les PKC phosphorylent des résidus sérine ou thréonine spécifiques sur des substrats protéiques, qui varient selon le type cellulaire. Dans de nombreuses cellules, l'activation des PKC augmente la transcription de gènes spécifiques.

Les protéines kinases C (PKC) sont des protéines codées par une famille de gènes (11 isoformes différentes). On sait notamment que ces protéines sont impliquées dans la transduction de signaux extracellulaires médiés par les facteurs de croissance, les cytokines, ainsi que par un certain nombre d'autres molécules biologiques. La protéine kinase β2 (PKC-β2) apparaît exprimée spécifiquement par les CL de l'épiderme.

La sphingosine est présente à l'état naturel dans la peau, et joue entre autre un rôle important dans fonction barrière du *stratum cornéum,* en tant que précurseur des sphingolipides (céramides et sphingoglycolipides). Elle peut être dérivée de source biologique telle que des extraits de cerveaux bovins ou par voie de synthèse, à partir de la serine, comme décrit par exemple dans l'article de Newman, J.Am. CHEM., 95 (12) : 4098 (1973). On peut plus particulièrement citer les formes isomères de la sphingosine : D-érythro, L-thréo, L-érythro et D-thréo. La forme D-erythro est la plus fréquemment présente dans la nature.

Les MMPs constituent une famille d'enzymes (actuellement plus d'une vingtaine ont été identifiées et caractérisées), zinc-dépendantes, de structure très conservée, qui possèdent la capacité de dégrader les composants de la matrice extracellulaire. Elles sont classées selon la nature de leur substrat en collagénases, gélatinases et stromélysine. Elles peuvent être synthétisées par différents types cellulaires au niveau de la peau (fibroblastes, kératinocytes, macrophages, cellules endothéliales, éosinophiles, cellules de Langerhans, etc.). Le groupe des MMPs est ainsi constitué de quatre sous-classes : (1) les collagénases, (2) les gélatinases, (3) les stromelysines et (4) les MMP de type membranaires (MT-MMPs). L'activité des MMPs peut être modulée par des inhibiteurs de protéinase naturellement présents tels que les inhibiteurs tissulaires de métalloprotéinases (TIMPs ; notamment les TIMP-1 et TIMP-2).

Le rôle prépondérant des MMPs dans le remodelage protéolytique de la matrice extracellulaire est maintenant clairement établi, à la fois dans des situations physiologiques (cicatrisation, angiogenèse, développement embryonnaire, etc.) et pathologiques (ulcère chronique, vieillissement cutané photo-induit, invasion des cellules tumorales, etc.).

Comme « composé inhibiteur des MMPs », en tant que composé actif pour inhiber la migration des cellules de Langerhans selon la présente invention, on entend les extraits peptidiques de lupin.

Le composé inhibiteur des MMPs selon la présente invention est choisi dans le groupe constitué par les extraits peptidiques de lupin ou « peptides de lupin », tels que ceux décrits dans la demande de brevet FR-99 04 875 déposée le 19 avril 1999 au nom de la société Laboratoires Pharmascience. On peut notamment citer l'extrait peptidique décrit dans la demande FR 99 04875 sous la dénomination extrait B (LU105).

Enfin, on a constaté de manière tout à fait surprenante et inattendue que l'on obtient des résultats particulièrement avantageux lorsque le composé actif pour inhiber la migration des cellules de Langerhans est une association d'au moins un composé inhibiteur des PKC, la sphingosine ou la phytosphingosine, avec au moins un composé inhibiteur des MMPs, un extrait peptidique de lupin.

On a ainsi pu constater, comme illustré dans les exemples ci-après, qu'une telle association spécifique permet avantageusement de potentialiser, voire de fournir un effet de synergie des activités respectives pour ainsi obtenir un effet spectaculaire d'inhibition de la migration des cellules de Langerhans, pouvant être assimilé à une annulation totale de la réponse immunitaire d'une peau réactive, sensible et/ou allergique, c'est à dire à l'obtention d'une réactivité d'une peau normale.

En particulier, la concentration en composé actif pour inhiber la migration des cellules de Langerhans est comprise entre 0,001 et 10% en poids, et plus particulièrement entre 0,01 et 3 % en poids, par rapport au poids total de la composition pharmaceutique ou cosmétique.

De préférence, la composition selon l'invention se caractérise en ce que le composé actif inhibiteur des PKC est la sphingosine et/ou ses isomères, et le dit composé inhibiteur des MMPs est un extrait peptidique de lupin, de manière plus préférée l'extrait B (LU 105).

La composition selon la présente invention peut en outre comprendre au moins un excipient pharmaceutiquement, notamment dermatologiquement, ou cosmétiquement acceptable. On peut utiliser tout excipient adapté pour les formes galéniques connues de l'homme de métier, en vue d'une administration par voie topique, orale, entérale ou parentérale.

En particulier, l'excipient peut être adapté pour l'obtention d'une composition sous forme d'une solution huileuse ou aqueuse, d'une émulsion eau-dans-huile, une émulsion huile-dans-eau, d'une microémulsion, d'un gel huileux ou aqueux, d'un gel anhydre, d'une crème, d'une lotion, d'un spray, d'un masque, d'un lait, d'une dispersion de vésicules, de microcapsules ou de microparticules, ou encore de gellules ou de capsules molles de gélatine ou végétales.

De préférence, on utilise un excipient adapté pour une administration par voie topique externe.

Enfin, la composition selon la présente invention peut en outre comprendre au moins un adjuvant pharmaceutiquement ou cosmétiquement acceptable connus de l'homme du métier, tels que des épaississants, des conservateurs, des parfums, des colorants, des filtres chimiques ou minéraux, des agents hydratants, des eaux thermales, etc.

La présente invention a également pour objet l'utilisation d'une association d'un composé actif inhibiteur des protéines kinases C (PKC) la sphingosine en la phytosphingosine et d'un composé actif inhibiteur des métalloprotéases matricielles (MMPs) un extrait peptidique de lupin pour la préparation d'une composition destinée à inhiber la migration des cellules de Langerhans.

La concentration en composé actif utilisé selon l'invention est comprise entre 0,001 et 10 % en poids, et plus particulièrement entre 0,01 et 3% en poids, par rapport au poids total de la composition pharmaceutique ou cosmétique.

La composition ainsi préparée peut en outre comprendre au moins un excipient pharmaceutiquement, notamment dermatologiquement, ou cosmétiquement acceptable, ainsi qu'au moins un adjuvant, tels que ceux décrits ci-dessus.

En particulier, la composition préparée par l'utilisation selon l'invention est destinée au traitement et à la prévention des réactions allergiques de la peau et des muqueuses (bouche, poumon, vessie, rectum, vagin), dans la mesure où elle permet de réduire une réponse allergique notamment induite par la migration de CL.

Plus particulièrement, la composition préparée par l'utilisation selon l'invention est destinée au traitement et à la prévention de l'eczéma atopique, dans la mesure où elle permet de réduire une réponse immunitaire notamment induite par la migration de CL ayant fixé des IgE en surface.

La composition préparée par l'utilisation selon l'invention est également destinée au traitement et à la prévention de l'eczéma de contact, dans la mesure où elle permet de réduire une réponse immunitaire notamment induite par capture d'un antigène, traitement et présentation de cet antigène aux lymphocytes T par les CL.

La composition préparée par l'utilisation selon l'invention est également destinée au traitement et à la prévention des peaux sensibles/réactives.

La composition préparée par l'utilisation selon l'invention est également destinée au traitement et à la prévention des dermatoses inflammatoires et/ou des dermites irritatives.

Il convient donc de noter que la composition décrite ci-dessus peut être avantageusement utilisée comme composants supplémentaire dans un produit pharmaceutique ou cosmétique ou encore un parfum, notamment à usage topique externe, contenant un composé actif principal présentant un caractère allergisant. La réaction allergique en sera ainsi avantageusement d'autant diminuée, voire la dose en composé actif principal présentant un caractère allergisant peut ainsi être avantageusement augmentée.

La composition préparée par l'utilisation selon l'invention est également destinée au traitement et à la prévention de la maladie auto-immune ou inflammatoire psoriasis.

La composition préparée par l'utilisation selon l'invention est également destinée à la prévention de la photoimmunosuppression.

Enfin, la composition préparée par l'utilisation selon l'invention est également destinée à la prévention du rejet de greffe.

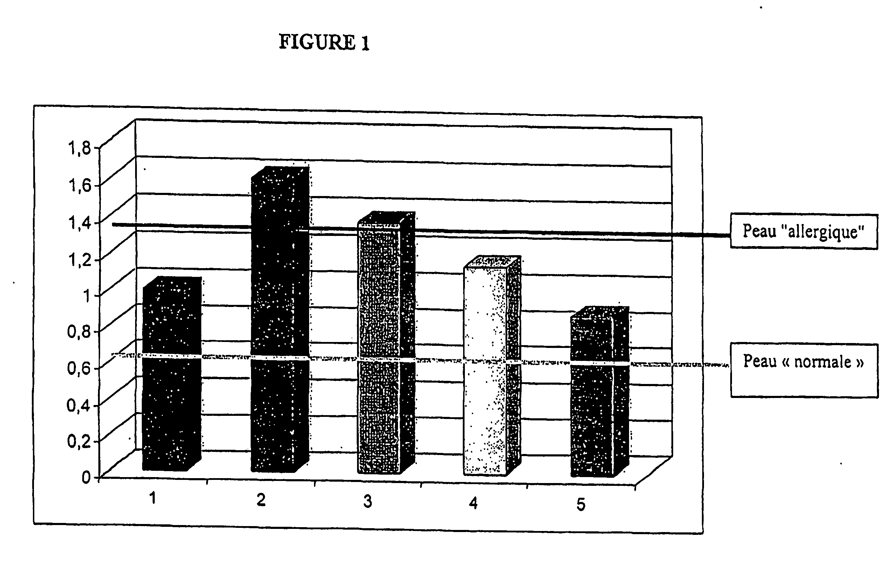

La Figure 1 est un histogramme illustrant les index migratoire des CL mesurés commedécrit dans l'exemple 1 : 1 : Cellules contrôles; 2 : Cellules sensibilisées par l'haptène DNSB; 3 : DNSB + LU 105 (5 µg/ml); 4 : DNSB + D-sphingosine (2,5 µM); 5 : DNSB + D-sphingosine (2,5 µM) + LU105 (5 µg/ml) (DNSB = dinitrosulphobenzène)

### Exemple 1 : étude de l'activité d'une association d'un inhibiteur des PKC avec un inhibiteur des MMPs sur l'inhibition de la migration des CL

### 1) Matériels & Méthodes

### 1.1 Obtention de suspension enrichies en CL

Des suspensions de cellules épidermiques ont été obtenues par traitement enzymatique (0,05% trypsine, pendant 18h à +4°C) de fragments de peau humaine normale issus de chirurgie plastique. Les suspensions obtenues contiennent en moyenne 2 à 4% de CL. L'obtention de suspensions contenant en moyenne 70% de CL, est basée sur le principe de la centrifugation sur gradient de densité (Lymphoprep™) et élimination des kératinocytes.

### 1.2 Préparation des milieux

Le milieu de base choisi pour l'ensemble de l'étude a été le RPMI 1640 (Gibco BRL, France).

De la D-sphingosine a été diluée dans de l'éthanol pour obtenir une solution mère à 5 x 10⁻³ M. La D-sphingosine a été utilisée à la concentration de 2,5 µM, dilution réalisée en RPMI contenant 1% de sérum albumine bovine (RPMI-BSA).

On a utilisé un extrait peptidique de lupin comme inhibiteur de MMPs. Il s'agit de l'extrait peptidique LU105 des Laboratoires Pharmascience. On a dilué LU105 dans du RPMI-1640 afin d'obtenir une solution mère à 250 mg/ml.

LU105 a été utilisé en final à la concentration de 5 µg/ml, dilution réalisée en RPMI-1640. Les cellules ont été préalablement incubées à 37°C pendant 60 min en présence de LU105 avant l'addition de l'haptène DNSB comme agent sensibilisateur dans le milieu.

### 1.3 Sensibilisation des CL

On a utilisé comme agent sensibilisateur le DNSB (Sigma Aldrich), forme soluble du DNCB (dinitro-chloro-benzène), a été solubilisé en RPMI-BSA et utilisé à la concentration de 50 µM.

### 1.4 Migration des CL

Un système de chambre de culture à deux compartiments (Falcon, Becton Dickinson, France) a été utilisé. Le compartiment supérieur est séparé du compartiment inférieur par une membrane de porosité 8 µm, sur laquelle sont déposées 50 µg/cm² de Matrigel. La membrane est alors recouverte de protéines formant un film équivalent à une membrane basale (laminine, collagène IV, nidogène, entactine, héparane sulfate protéoglycanes). Les cellules reprises dans le milieu RPMI-BSA seul ou en présence des différents produits sont déposées dans le compartiment supérieur. Dans le compartiment inférieur, est ajouté du surnageant de culture de fibroblastes humains normaux. Après 18h d'incubation à 37°C, le nombre de cellules vivantes ayant traversé le Matrigel et se trouvant dans le compartiment inférieur est compté sous microscope (les CL sont facilement identifiables par leur forme dendritique). Chaque essai est réalisé en triplicate.

### 2) Résultats

### 2.1 Les résultats sont présentés dans le tableau 3 suivant et illustrés par l'histogramme de là Figure 1.

**Tableau 3 : index de migration des CL**

| | 1 | 2 | 3 | 4 | 5 |
|---|---|---|---|---|---|
| index de migration | 1 | 1,61 | 1,38 | 1,15 | 0,88 |

| | | | | | |
|---|---|---|---|---|---|
| *Légende tableau 3 et de l'histogramme de la figure 1* | | | | | |
| 1 : Cellules contrôles | | | | | |
| 2 : Cellules sensibilisées par l'haptène DNSB | | | | | |
| 3 : DNSB + LU105 (5 µg/ml) | | | | | |
| 4 : DNSB + D-sphingosine (2,5 µM) | | | | | |
| 5 : DNSB + LU105 (5 µg/ml) + D-sphingosine (2,5 µM) | | | | | |

### 2.2 Migration des CL

Les résultats représentent le rapport entre le nombre de cellules ayant migré en présence de DNSB +/- D-sphingosine ou LU105 ou l'association D-sphingosine + LU105 et le nombre de cellules ayant migré dans les conditions normales (cellules contrôles non sensibilisées et non traitées) . Les CL fraîchement isolées de l'épiderme n'ont pas une capacité migratoire élevée. Dans l'expression des résultats, la capacité migratoire des CL contrôles (non traitées et non sensibilisées) est arbitrairement fixée à 1.

Le traitement des cellules avec l'haptène DNSB a stimulé la migration des CL de façon significative (augmentation de 61%) par rapport aux cellules normales non stimulées (cellules contrôles).

La D-sphingosine aux concentrations de 2,5 µM (tableau 3) inhibe de manière significative la migration des CL induite par le DNSB.

Dans une expérience où les cellules n'ont pas été mises en contact avec l'haptène DNSB, mais avec LU 105 seul (trois concentrations), aucune modification de l'index de migration par rapport aux cellules contrôles (non sensibilisées et non traitées) n'a été observée. Ceci indique qu'en absence de stimulation des CL, LU105 n'a pas d'effet sur la migration. LU105 (5 µg/ml) inhibe de manière significative la migration des CL induite par le DNSB.

L'association D-sphingosine +LU105 permet d'inhiber totalement l'effet du DNSB sur les CL, le nombre de CL ayant migré en présence de DNSB + D-sphingosine + LU105 étant similaire à celui des cellules contrôles (non sensibilisées et non traitées). Cette association a donc un effet potentialisateur sur les propriétés intrinsèques des deux produits testés.

### 3) Conclusions

Dans ce travail, nous avons démontré en utilisant des CL fraîchement isolées, placées dans un système de chambre de culture à deux compartiments (permettant la migration cellulaire), que la D-sphingosine (inhibiteur des PKC) potentialise J'effet inhibiteur du LU105 (inhibiteur de MMPs) sur la migration des CL sensibilisées par l'haptène DNSB, et inversement. En effet, ces deux molécules utilisées séparément inhibent de manière significative la migration des CL. Lorsqu'elles sont associées les CL sensibilisées ont une capacité migratoire semblable à celle des CL non activées. En d'autres termes, d'après cet exemple, l'association d'un inhibiteur de PKC et d'un inhibiteur de MMPs permet d'annuler totalement la réponse immunitaire des peaux réactives/sensibles et allergiques, et donc de retrouver une peau normale.

## Revendications

1. Composition pharmaceutique ou cosmétique, **caractérisée en ce qu'**elle comprend une association
■ d'un composé actif pour inhiber la migration des cellules de Langerhans et inhibiteur des PKC qui est la sphingosine ou la phytosphingosine et
■ d'un composé actif pour inhiber la migration des cellules de Langerhans et inhibiteur des MMPs qui est un extrait peptidique de lupin
et au moins un excipient pharmaceutiquement ou cosmétiquement acceptable.

2. Composition selon la revendication 1, **caractérisée en ce que** la concentration en composé actif pour inhiber la migration des cellules de Langerhans est comprise entre 0,001 et 10% en poids, par rapport au poids total de la composition pharmaceutique ou cosmétique.

3. Utilisation d'une composition selon la revendication 1 pour préparer un médicament pour inhiber l'induction ou la propagation de la réponse immune et/ou inflammatoire, à savoir pour le traitement et à la prévention des réactions allergiques de la peau et des muqueuses.

4. Utilisation d'une composition selon la revendication 1 pour préparer un médicament pour inhiber l'induction ou la propagation de la réponse immune et/ou inflammatoire, à savoir pour le traitement et à la prévention de l'eczéma atopique.

5. Utilisation d'une composition selon la revendication 1 pour préparer un médicament pour inhiber l'induction ou la propagation de la réponse immune et/ou inflammatoire, à savoir pour le traitement et à la prévention de l'eczéma de contact.

6. Utilisation d'une composition selon la revendication 1 pour préparer un médicament pour inhiber l'induction ou la propagation de la réponse immune et/ou inflammatoire, à savoir pour le traitement et la prévention des peaux sensibles/réactives.

7. Utilisation d'une composition selon la revendication 1 pour préparer un médicament pour inhiber l'induction ou la propagation de la réponse immune et/ou inflammatoire, à savoir pour le traitement et la prévention des dermatoses inflammatoires.

8. Utilisation d'une composition selon la revendication 1 pour préparer un médicament pour inhiber l'induction ou la propagation de la réponse immune et/ou inflammatoire, à savoir pour le traitement et la prévention des dermites irritatives.

9. Utilisation d'une composition selon la revendication 1 pour préparer un médicament pour inhiber l'induction ou la propagation de la réponse immune et/ou inflammatoire, à savoir pour la prévention du rejet de greffe.

10. Utilisation d'une composition selon la revendication 1 pour préparer un médicament pour inhiber l'induction ou la propagation de la réponse immune et/ou inflammatoire, à savoir pour diminuer le caractère allergisant d'une préparation cosmétique ou pharmaceutique, ou d'un parfum.

11. Utilisation d'une composition selon la revendication 1 pour préparer un médicament pour inhiber l'induction ou la propagation de la réponse immune et/ou inflammatoire, à savoir pour le traitement et la prévention de la maladie auto-immune ou inflammatoire psoriasis, ou à la prévention de la photoimmuno suppression.

## Claims

1. Pharmaceutical or cosmetic composition, **characterized in that** it comprises a combination
• of an active compound for inhibiting Langerhans cell migration and which is a PKC inhibitor, which compound is sphingosine or phytosphingosine, and
• of an active compound for inhibiting Langerhans cell migration and which is an MMP inhibitor, which compound is a peptide extract of lupin,
and at least one pharmaceutically or cosmetically acceptable excipient.

2. Composition according to Claim 1, **characterized in that** the concentration of active compound for inhibiting Langerhans cell migration is between 0.001 and 10% by weight, relative to the total weight of the pharmaceutical or cosmetic composition.

3. Use of a composition according to Claim 1, for preparing a medicinal product for inhibiting the induction or the propagation of the immune and/or inflammatory response, namely for the treatment and prevention of allergic reactions of the skin and the mucous membranes.

4. Use of a composition according to Claim 1, for preparing a medicinal product for inhibiting the induction or the propagation of the immune and/or inflammatory response, namely for the treatment and prevention of atopic eczema.

5. Use of a composition according to Claim 1, for preparing a medicinal product for inhibiting the induction or the propagation of the immune and/or inflammatory response, namely for the treatment and prevention of contact eczema.

6. Use of a composition according to Claim 1, for preparing a medicinal product for inhibiting the induction or the propagation of the immune and/or inflammatory response, namely for the treatment and prevention of sensitive/reactive skin.

7. Use of a composition according to Claim 1, for preparing a medicinal product for inhibiting the induction or the propagation of the immune and/or inflammatory response, namely for the treatment and prevention of inflammatory dermatosis.

8. Use of a composition according to Claim 1, for preparing a medicinal product for inhibiting the induction or the propagation of the immune and/or inflammatory response, namely for the treatment and prevention of irritant dermatitis.

9. Use of a composition according to Claim 1, for preparing a medicinal product for inhibiting the induction or the propagation of the immune and/or inflammatory response, namely for the prevention of transplant rejection.

10. Use of a composition according to Claim 1, for preparing a medicinal product for inhibiting the induction or the propagation of the immune and/or inflammatory response, namely for decreasing the allergenic nature of a cosmetic or pharmaceutical preparation, or of a fragrance.

11. Use of a composition according to Claim 1, for preparing a medicinal product for inhibiting the induction or the propagation of the immune and/or inflammatory response, namely for the treatment and prevention of the autoimmune or inflammatory disease psoriasis, or for the prevention of photoimmunosuppression.

## Patentansprüche

1. Pharmazeutische oder kosmetische Zusammensetzung, **dadurch gekennzeichnet, dass** sie eine Kombination
• einer Verbindung, welche wirksam ist, um die Migration der Langerhans'-Zellen zu hemmen, und Inhibitor der PKC, welche Sphingosin oder Phytosphingosin ist, und
• einer Verbindung, welche wirksam ist, um die Migration der Langerhans'-Zellen zu hemmen, und Inhibitor der MMPs, welche ein peptidischer Extrakt von Lupine ist, und wenigstens einen aus pharmazeutischer oder kosmetischer Sicht annehmbaren Träger umfasst.

2. Zusammensetzung nach Anspruch 1, **dadurch gekennzeichnet, dass** die Konzentration an Verbindung, welche wirksam ist, um die Migration der Langerhans'-Zellen zu hemmen, zwischen 0,001 und 10 Gew.-% eingeschlossen bezogen auf das Gesamtgewicht der pharmazeutischen oder kosmetischen Zusammensetzung liegt.

3. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung von allergischen Reaktionen der Haut und der Schleimhäute.

4. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung des atopischen Ekzems.

5. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung des Kontaktekzems.

6. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung von empfindlicher/reaktiver Haut.

7. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung von entzündlichen Dermatosen.

8. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung von irritativen Dermitiden.

9. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Verhütung von Transplantatabstoßung.

10. Verwendung einer Zusammensetzung nach Anspruch 1 für das Herstellen eines Arzneimittels, um die Induktion oder die Ausweitung der Immun- und/oder Entzündungsantwort zu hemmen, nämlich um den allergisierenden Charakter einer kosmetischen oder pharmazeutischen Zubereitung oder eines Parfums zu verringern.

11. Verwendung einer Zusammensetzung nach Anspruch 1 für das Herstellen eines Arzneimittels, um die Induktion oder die Ausweitung der Immun- und/oder Entzündungsantwort zu hemmen, nämlich für die Behandlung und die Verhütung der Autoimmun- oder Entzündungserkrankung Psoriasis oder für die Verhütung der Photoimmunosuppression.
